Europäisches Patentamt

**European Patent Office**

**Office européen des brevets**

(19)

(11) Publication number: **0 173 295**
A1

(12) # EUROPEAN PATENT APPLICATION

(21) Application number: **85110742.5**

(22) Date of filing: **27.08.85**

(51) Int. Cl.⁴: **G 01 N 33/53, G 01 N 33/576**

(30) Priority: **31.08.84 US 646658**

(43) Date of publication of application: **05.03.86**
Bulletin 86/10

(84) Designated Contracting States: **AT BE CH DE FR GB IT
LI NL SE**

(71) Applicant: **New York Blood Center, Inc.,
310 East 67 Street, New York, New York 10021 (US)**

(72) Inventor: **Prince, Alfred M., Stone Hill Road, Pound Ridge
New York 10576 (US)**
Inventor: **Woods, Kenneth R., 382 Carpenter Avenue,
Sea Cliff New York 11579 (US)**
Inventor: **Wiebe, Michel E., 83 Haviland Court, Stamford
Connecticut 06903 (US)**

(74) Representative: **Eggert, Hans-Gunther, Dr.,
Räderscheidtstrasse 1, D-5000 Köln 41 (DE)**

(54) **Assay for simultaneous detection of antigen and antibody in given serum.**

(57) A process is disclosed for the simultaneous detection of the presence of either or both of an antigen or an antibody in a single test specimen. The described process requires a single solid support for an immunological assay which screens simultaneously for an antigen and an antibody and, therefore, minimizes the number of tests required for screening a given body fluid or tissue.

EP 0 173 295 A1

## BACKGROUND OF THE INVENTION

### Field of the Invention

This invention relates to a process for the screening of body fluids and tissues to determine if the same contain any of a plurality of immunological markers. More especially, this invention relates to a screening process which detects simultaneously if a given sample contains either a given antigen or a given antibody of a species other than that of said antigen. Still more specifically, this invention relates to such a screening process which in a single test procedure using a single test zone determines if any of a plurality of immunological markers comprising a mixture of an antigen of one species and an antibody if another species is present.

### Discussion of the Prior Art

Immunologic techniques have been widely exploited to specifically probe for particular antigens or antibodies in biological tissues and fluids. Tests based on immunologic properties of reactants can be made to be highly specific and sensitive. They all involve two fundamental steps; 1) bringing together antigen and antibody moieties under conditions that enable immune complex formation to occur, and 2) employing means to detect the presence and extent of such product formation. While simple in principle, selection and development of reliable immunologic and detection reagents and means for their disposition in workable proportions and separation from extraneous materials, as well as the terminology often used to describe particular systems, may become complex and confusing.

Tests have been devised and widely used to detect the presence of antigens associated with certain pathogens, for example immunologic detection of hepatitis B surface antigen has been associated with the presence of hepatitis B virus in human blood specimens capable of transmitting the disease.

Antibodies may also be used as markers of pathogens in human blood. Tests designed to exploit antibody markers currently in use or being developed include antibody to hepatitis B virus core antigen, antibody to cytomegalo virus, antibody Treponenia pallidum (Syphilis), antibody to Malaria and Chagas disease, antibodies to human T cell lymphotropic virus or (HTLV) strains I, II and III, or lymphadenopathy associated (LAV) virus.

HTLV-III and LAV viruses may be identical or equivalent. Since there is a very much higher frequency of HTLV-III or LAV antibody in the blood of pre-AIDS patients than in healthy persons, it is now considered likely that these are the pathogens primarily responsible for induction and transmission of the acquired immunodeficiency syndrome (AIDS). Such antibodies indicate previous exposure of individuals to particular pathogens and in the case of AIDS suggest that patients are hosts to residual infectious HTLV-III (LAV) virus that may be transmitted to others by sexual contact or blood transfusions.

Additional infectious agents that underlie significant risk of disease transmission by venereal or blood vectors may be isolated and characterized in the future. It is suspected but not yet proven that Alzheimer's Disease and Creutzfeldt-Jacob Disease may be caused by such agents.

A third category of potentially useful markers of pathogens that may be detected by immunochemical probes includes enzymes or other specific proteins arising from cytopathology induced by pathogen invasion of body tissues. For example, the liver derived enzyme alanine amino transfereses (ALT) often exceeds normal levels in the blood of persons having viral hepatitis non A, non B.

Batteries of immunologic tests for markers of pathogens can be assembled to test for their presence at detectable levels in the blood and tissues intended for transfusion or transplantation. Tests thus far developed are designed to deliver samples of a biological specimen to zones or containers where a single specific immunologic reagent has been disposed in such a manner as to be capable of binding the particular biochemical marker (antigen, antibody, or enzyme) being probed.

Thus far, such tests have only been able to detect one marker in each test zone. This approach will become increasingly time consuming and expensive as each new test is developed and added to the battery of probes used for diagnosis of illness or used to screen blood and blood products intended for transfusion or tissues intended for transplantation.

One example of a test battery is the "Torch" kit in which immunologic reagents are disposed within uniform depressions in a plastic "micro titre tray". The tray is arranged to provide means to test for more than one marker, but only one marker per test zone, with different zones for binding and detection of the different markers.

It has, therefore, become desirable to minimize the number of tests to be conducted while confirming the presence or absence of as many immunological markers (antigens and antibodies) as possible. In other words, it has become desirable, indeed almost mandatory, to screen a body of fluid or tissue for as many markers of different pathogens while minimizing the steps required for such purpose. Indeed, it has become critical to minimize the costs attendant screening processes without compromising the scope of the screening procedure.

## SUMMARY OF THE INVENTION

In accordance with the aforestated objects, this invention broadly contemplates a process for the detection of the presence of either a first antigen of a first pathogen or a first antibody of a second pathogen in a test specimen which comprises

A) passing said test specimen over solid support having affixed thereto an antibody to said first antigen and an antigen to said first antibody;

4

B)   Passing over the resultant composition of step A a second antibody to said first antigen which second antibody is of a different animal species and an antibody to said first antibody of a different animal species;

C)   immunologically detecting the presence of said second antibody to said first antigen and said antibody to said first antibody.

Step C can be performed using a radioisotope or enzyme labelled antibody of a species different from the species of (a) said second antibody to said first antigen and said antibody to said first antibody. Once the radioisotope or enzyme labelled antibody has formed a complex with said second antibody to said first antigen and/or said antibody to said first antibody detection is singly a matter or determining the radioactivity or enzyme activity in the normal manner and comparing it against a control. A higher radioactivity (number of counts) or enzyme activity confirms that either or both of the first antigen and/or first antibody are/is present in the test specimen.

It is to be understood that the process is usually performed by washing and incubating in the usual way after each antigen and/or antibody containing mass is passed over the solid support.

The third step of the process can be performed in a single step or as a series of steps and the choice of the number of steps depends upon the nature of the second antibody to the first antigen and the antibody to the first antibody as well as the availability of reagents. If the second antibody to the first antigen and the antibody to the first antibody are of the same species, then the third step can be performed in a single step using radioisotope or enzyme labelled antibody thereto of another species.

Where, however, the second antibody to the first antigen is of a species different from that of the antibody to the first antibody, it will normally by necessary to detect the presence of these antibodies serially by using radioisotope or enzyme labelled antibodies of different species.

The process can be more easily understood by the following narrative example. A typical solid support of the type known for use in immunoassays is obtained. To that support is affixed an antibody of the antigen to be detected and an antigen to the antibody to be detected. The test specimen is passed thereover, incubated and washed. If either or both of the antigens and/or antibody to be probed is present in the test specimen, it binds to the antibody or antigen prefixed to the solid support.

Thereafter, an antibody to the antigen being probed and an antibody to the antibody being probed are passed over the solid support. Here it is necessary to use

6

an antibody of a species different from the antibody to the antigen initially affixed to the solid support. Similarly, the antibody to the antibody being probed must be of a species different from the antibody being probed, but can be and in part is desirably of the same species as the antibody used to bind to the probed antigen, i.e., the so called second antigen. Following washing and incubating, the presence of these last used antibodies is detected.

The table below illustrates a typically combined antigen/antibody assay according to the invention using lentil lectin as a model antigen and mouse anti-fibrin as a model antibody.

<u>COMBINED ANTIGEN/ANTIBODY ASSAY</u>

SWINE ANTI-RABBIT IG-E*

RABBIT ANTI-LENTIL + RABBIT ANTI-MOUSE IG LENTIL LECTIN + MOUSE ANTI-FIBRIN MCAB GOAT ANTI-LENTIL + FIBRIN

=============================================

Goat anti-lentil is used as the antibody to the first antigen which fibrin, an antigen, is used as antigen for the mouse anti-fibrin being probed. After the goat anti-lentil and fibrin have been fixed to the solid support, the test specimen suspected of continuing lentil lectin and/or mouse anti-fibrin is passed over the support, thereby causing immune complex formation when there probes are present. Following washing and incubation, the solid support

7

bearing the immune complex is treated with rabbit anti-lentil and rabbit anti-mouse IG, the former an antibody to the antigen being probed and the latter being an antibody to the antibody being probed. These latter reagents bind to antigen and antibody being probed if present in the immune complex. Swine anti-rabbit IG which has been tagged with an enzyme can then be used to bind immunologically to either or both of the rabbit anti-lentil or rabbit anti-mouse antibody if either are present. Detection of higher enzyme activity is proof that either or both of the lentil lectin or/and mouse anti-fibrin is/are present in the test specimen.

The process is useful for the simultaneous screening of additional pathogen markers. For instance, the process can be used to screen for two or more antigens and at least one antibody or two or more antibodies and at least one antigen. In fact, the process can screen for two or more antigens and two or more antibodies simultaneously using a single solid support.

In the case where the process is employed to screen for two antigens and an antibody, the solid support has affixed thereto an additional antibody which is an antibody to the second antigen being probed. After the test specimen is passed over the solid support bearing the two different antibodies (antibodies to different antigens) and the antigen, the second step of the process is performed as described above, but there is additionally added an antibody to said second antigen being probed differently in respect of its species from that affixed to the solid support. The final step can be performed in the same manner or described above.

The present invention, therefore, provides means whereby mixtures of reagents capable of binding markers of a variety of pathogens are prepared in suitable proportions and disposed together within each test zone in such a manner that the presence of any one or more pathogen markers contained in a specimen may be bound to the reagent so disposed upon the matrix in sufficient quantity as to be detectable by mixtures of immunologic, enzymatic radio-metric or other detection reagents. (Alternatively, reagents capable of binding markers of a variety of pathogens or other antigens, antibodies or enzymes may be serially disposed within each zone.) This invention thereby enables low frequency markers to be probed for collaterally within a single specimen delivered to the test zone. Identification of a particular pathogen, antigen, antibody or enzyme marker or combinations thereof when encountered can be discriminated by simply retesting each specimen separately, using the conventional one test zone-one specimen marker immunoassay technique.

The challenging and unique feature of this development has been to devise means to test simultaneously for several marker categories, whether antigen or antibody or enzyme, or mixtures thereof, never heretofore conceived or accomplished to the best of our knowledge.

## DESCRIPTION OF SPECIFIC EMBODIMENTS

The general procedure of the invention is as follows:

1. A mixture of antibodies to the pathogens or other immunogens to be detected, and antigens for the antibodies to be detected are comingled and immobilized on a solid matrix.

2. Body fluids which may contain pathogens or other immunogens and/or antibodies to be detected are placed in contact with the immobilized reagents. Specificity for the immobilized reagents results in selective binding and immobilization from the body fluid of the antigens and/or antibodies being probed for.

3. Bound antigens and/or antibodies from the body fluids may then be detected by interaction with a mixture of immunologically specific reagents, each specific for one of the immobilized antigens or antibodies. Interaction with immobilized body fluid antigens and/or antibodies results in the immobilization of the immunologically specific reagents.

4. The immobilized, immunologically specific reagents can be detected directly if they are tagged (for example, with an isotope or enzyme). They can be detected indirectly with tagged antibodies which specifically bind to them.

The invention is, therefore, a process verifying the presence of a first antibody and/or a first antigen in a test specimen which comprises the steps of

A. disposing a second antibody of said first antigen and second antigen of said first antibody on a solid support;

B. passing said test specimen over said solid support bearing said second antibody and said second antigen whereby said first antigen and said first antibody in said test specimen become bound to second antibody and second antigen;

C. either

1) contacting the resultant mass from step B with a mixture of a third antibody which is an antibody to said first antibody and a fourth which is an antibody to said first antigen said third and fourth antibodies bearing markers for analysis and analyzing for the presence of said third or fourth or both antibodies.

2) contacting the resultant mass from step B with a mixture of a third antibody which is an antibody to said first antigen, thereafter contacting the resultant mass with a mixture of a marked fifth, antibody and said third antibody and a sixth antibody to said fourth antibody.

The fifth and sixth antibody can be the same when the third and fourth antibodies are from the same animal species.

Using the above mode of describing the invention, the table below illustrates typical ways for carrying out screening according to the invention for various combinations of antigens and antibodies.

In order to more fully illustrate the nature of the invention and the manner of practicing the same, the following examples are presented.

Example 1

Immunoassay for Mixtures of Lentil (Lens culinaris) Lectin Antigen and Antibody to Human Fibrin

A mixture of goat anti-lentil lectin antibody (Accurate Chemical, 200 ng) and human fibrin (800 ng IMCO) in 0.015 M $Na_2CO_3$; 0.035 M $NaHCO_3$, pH 9.6 was adsorbed to the surface of each well in a 96-well, polyvinyl microtiter tray (Costar) during 2 hours of incubation at ambient

12

temperature. Coated wells were washed three times with phosphate buffered saline (PBS), pH 7.4 containing 0.05% (v/v) Tween-20 detergent. Bovine serum albumin (Sigma, RIA grade, 2 mg/well) in PBS, pH 7.4 was added to each well and incubated for 15 minutes at ambient temperature to block all unbound sites. Coated and blocked wells were washed three times with Tween/PBS as stated above.

Test mixtures of mouse monoclonal antibody to human fibrin (T2G1$_s$, Kudryk, B. et al. 1984. Molec. Immun. 20: 1191-1200), and lentil lectin (prepared at New York Blood Center) were constructed at various ratios as detailed in Table 1. 100 uL of each mixture was added to a coated microtiter well and incubated for 15 minutes at ambient temperature. The test mixtures were removed and the wells washed threee times with Tween/PBS as stated above. A 100 uL mixture of rabbit anti-mouse Ig (Accurate Chemical, 1:100 dilution) and rabbit anti-lentil lectin antibody (prepared at New York Blood Center, 1:50 dilution) was added to each well and incubated for 15 minutes at ambient temperature.

The unbound rabbit antibody was removed and each well washed three times with Tween/PBS as stated above. 100 uL of horse radish peroxidase-linked swine anti-rabbit Ig (Accurate Chemical, 1:100) was added to each well and incubated for 15 minutes at ambient temperature. The unbound swine antibody was removed and each well washed three times with Tween/PBS as stated above.

100 uL of O-Dianisidine Di HCl, 1 mg/mL, in 0.3% $H_2O_2$, (Sigma) was added to each well and incubated for 15 minutes at ambient temperature. The enzyme reaction was terminated by the addition of 100 uL of 0.1 N HCl to each well. Absorbance was quantitated for each well by using the MR 580 Microelisa Autoreader (Dynatech, Alexandria, VA). Results are shown in Table 2 below.

### TABLE 1

| Lentil Lectin (ng/mL) | Anti-fibrin Antibody (ug/mL) |
|---|---|
| 0 | 15.00 |
| 0 | 7.50 |
| 0 | 3.75 |
| 0 | 1.87 |
| 0 | 0 |
| 125 | 15.00 |
| 125 | 7.50 |
| 125 | 3.75 |
| 125 | 1.87 |
| 125 | 0 |
| 250 | 15.00 |
| 250 | 7.50 |
| 250 | 3.75 |
| 250 | 1.87 |

14

TABLE 1 (cont'd)

| Lentil Lectin (ng/mL) | Anti-fibrin Antibody (ug/mL) |
|---|---|
| 250 | 0 |
| 500 | 15.00 |
| 500 | 7.50 |
| 500 | 3.75 |
| 500 | 1.87 |
| 500 | 0 |

TABLE 2

RESULTS

Immunoassay of Mixtures of Antigen and Antibody*

| Lentil Lectin (ng/mL) | Anti-Fibrin Antibody (ug/mL) | | | | |
|---|---|---|---|---|---|
| | 0 | 1.87 | 3.75 | 7.50 | 15.00 |
| 0 | 0.00 | 0.06 | 0.11 | 0.20 | 0.37 |
| 125 | 0.21 | 0.51 | 0.58 | 0.65 | 0.66 |
| 250 | 0.32 | 0.83 | 0.77 | 0.78 | 0.83 |
| 500 | 0.52 | 0.88 | 0.90 | 0.90 | 0.89 |

*Results expressed as optical density in ELISA assay.

Example 2

Simultaneous Detection of Ant. HTLV III and HBsAG

500 ng of purified HTLV-III disrupted virions, and 1 ugm of Polyclonal anti-HBs prepared by immunization of guinea pigs, are adsorbed to the surface of microtiter plates in 200 ul of carbonate buffer pH 9.6 for 18 hours at room temperature. The wells are then filled with buffered saline pH 8.0 containing 5 mg/ml of bovine serum albuim and 1% normal human serum, 1% normal rabbit serum and 1% normal mouse serum to block nonspecific adsorption of the subsequently used reagents, and incubated for one hour at 20°C. After three washes with distilled $H_2O$, the plates are dried and stored at 4°C. This constitutes the "capture system".

Sera from patients with AIDS syndrome, containing antibody to HTLV-III antigens; or sera from carriers of hepatitis B virus, containing HBsAg, or normal sera lacking both reactants, are adsorbed to the above microtiter wells for two hours at 45°C. The plates are then washed three times with distilled water.

Detection probes to bind the target antigens and antibodies are then added. These consist of a biotinylated mouse monoclonal antibody against HBsAg and biotinylated rabbit $F(ab^1)2$ fragments of rabbit antibodies to human IgG and IgM (to detect bound anti-HTLV-III). After incubation for one hour at 45°C and washing with distilled water a

16

Steptavidin - biotin - horse radish peroxidase conjugate (obtained from Bethesda Research Labs, Bethesda, Maryland) is added and the plates are further incubated for 30 minutes at room temperature. Finally, a substrate solution is added and the plates are incubated for 30 minutes to permit color development. The reaction is stopped with 1M $H_2SO_4$ and the optical densities are read on a microplate reader.

As shown in Table 3 below, the test is able to detect either HBsAg or anti-HTLV-III.

Specificity can subsequently be determined by neutralizing reactants with an excess of either HTLV-III antigen or anti-HBs antibody.

TABLE 3

RESULTS OF TESTS ON AIDS AND HBV CARRIER SERUM

| Serum | Optical Density Reading | Conclusion |
|-------|-------------------------|------------|
| AIDS-I | 0.34 | + |
| AIDS-2 | 0.61 | + |
| AIDS-3 | 0.21 | + |
| HBV Carrier-1 | 1.8 | + |
| HBV Carrier-2 | 2.1 | + |
| HBV Carrier-3 | 1.9 | + |
| Control Normal 1 | 0.01 | - ↓ |
| Control Normal 2 | 0.03 | - |
| Control Normal 3 | 0.02 | - |

↓ Upper Limit of Normal = 3x negative control mean values = 0.06.

WHAT IS CLAIMED IS:

1.   A process for the detection of the presence of either a first antigen of a first pathogen or a first antigen of a second pathogen in a test specimen which comprises:

A)   passing said tests specimen over a solid support having affixed thereto an antibody to said first antigen and an antigen to said first antibody;

B)   passing over the resultant composition of step a, a second antibody to said first antigen which second antibody is of a different animal species and an antibody to said first antibody of a different animal species;

C) immunologically detecting the presence of said second antibody to said first antigen and said antibody to said first antibody.

2.   A process according to claim 1 wherein said first antigen is human hepatitis B virus surface antigen.

3.   A process according to claim 1 wherein said first antibody is human anti-HTLV-III antibody.

4.   A process according to claim 3 wherein said first antigen is human hepatitis B surface antigen.

5.   A process according to claim 1 wherein said first antibody is human anti-cytomegalo virus antibody.

6.   A process according to claim 5 wherein said first antigen is human hepatitis B surface antigen.

7.   A process for verifying the presence of a first antibody and/or a first antigen in a test specimen

18

which comprises the steps of:

A) disposing a second antibody of said first antigen and a second antigen of said first antibody on a solid support;

B) passing said test specimen over said solid support bearing said second antibody and said second antigen whereby first antigen and first antibody in said test specimen become bound to said second antibody and second antigen, respectively;

C) either

1) contacting the resultant mass from step b with a mixture of a third antibody which is a antibody to said first antibody and a fourth antibody which is an antibody to said first antigen, said third and fourth antibodies bearing markers for analysis and analyzing for the presence of said third or fourth or both antibodies;

2) contacting the resultant mass from step b with a mixture of a third antibody which is an antibody to said first antibody and a fourth antibody which is an antibody to said first antigen, thereafter contacting the resultant mass with a mixture of a marker fifth antibody to said third antibody and a marked sixth antibody to said fourth antibody.

8. A process according to claim 7 wherein step c is carried out by contacting the resultant mass from step b with a mixture of a third antibody which is an antibody to said first antibody and a fourth antibody which is an antibody to said first antigen, said third and fourth antibodies bearing markers for analysis and analyzing for

19

the presence of said third or fourth or both antibodies.

9.    A process according claim 7 wherein step c is conducting by contacting the resultant mass from step b with a mixture of a third antibody which is an antibody to said first antibody and a fourth antibody which is an antibody to said first antigen, thereafter contacting the resultant mass with a mixture of a marked fifth antibody to said third antibody and a sixth antibody to said fourth antibody.

10.    A process according to claim 9 wherein said third and fourth antibodies are from the same animal species and said fifth and sixth antibodies are the same as one another.

11.    A process according to claim 7 wherein said first antibody is human anti-HTLV-III antibody.

12.    A process according to claim 7 wherein said first antigen is hepatitis B surface antigen.

13.    A process according to claim 12 wherein said first antibody is human HTLV-III antibody.

14.    A process according to claim 12 wherein said antibody is human anti-cytomegalo virus antibody.

15.    A process according to claim 1 wherein step c is performed using an enzyme labelled immunoassay.

16.    A process according to claim 1 wherein step c is performed using a radio immunoassay.

17.    A process according to claim 7 wherein step c is performed using a third and fourth antibodies bearing enzymes markers for analysis.

18.    A process according to claim 8 wherein said third and fourth antibodies their radio-active isotopes as markers.

19. A process according to claim 9 wherein said fifth and sixth antibodies are marked with an enzyme detectable by enzyme immunoassay.

20. A process according to claim 9 wherein said fifth and sixth antibodies are marked with a radioactive isotope detectable by radio immunoassay.

21

0 173 295

**EUROPEAN SEARCH REPORT**

**European Patent Office**

| | DOCUMENTS CONSIDERED TO BE RELEVANT | | EP 85110742.5 |
|---|---|---|---|

| Category | Citation of document with indication. where appropriate, of relevant passages | Relevant to claim | CLASSIFICATION OF THE APPLICATION (Int. Cl 4) |
|---|---|---|---|
| A | EP - A1 - 0 019 277 (NEW YORK BLOOD CENTER) <br> * Claims * <br> -- | 1,6,7, 15,16 | G 01 N 33/53 <br> G 01 N 33/576 |
| A | US - A - 4 242 322 (SEAN P. O'NEILL) <br> * Column 3, line 63 - column 4, line 2; claims * <br> -- | 1,2,4, 6 | |
| A | US - A - 3 872 225 (J.A. COLLER et al.) <br> * Claims * <br> -- | 1,7,16 | |
| A | EP - A1 - 0 083 869 (INTERNATIONAL INSTITUTE OF CELLULAR AND MOLECULAR PATHOLOGY) <br> * Pages 2-5 * <br> ---- | 1 | |

| | | | TECHNICAL FIELDS SEARCHED (Int. Cl 4) |
|---|---|---|---|
| | | | G 01 N 33/00 |

The present search report has been drawn up for all claims

| Place of search | Date of completion of the search | Examiner |
|---|---|---|
| VIENNA | 29-11-1985 | IRMLER |